# EUROPÄISCHE PATENTANMELDUNG

(11) **EP 4 475 564 A1**
(43) Veröffentlichungstag der Anmeldung: **11.12.2024**
(21) Anmeldenummer: 24177758.0
(22) Anmeldetag: 23.05.2024
(51) Int. Cl.: H04R 25/00, H04S 7/00, H04H 20/61

(54) **BETRIEB EINER HÖRVORRICHTUNG IN EINEM AUDIODARBIETUNGSSYSTEM**

(30) Priorität: 26.05.2023 DE 102023204982
(71) Anmelder: Sivantos Pte. Ltd., Singapore 539775 (SG)
(72) Erfinder: MITRA, Subhasri, 91058 Erlangen (DE); PFROMMER, Andreas, 91058 Erlangen (DE)
(74) Vertreter: FDST Patentanwälte

(57) **Zusammenfassung**

Es wird ein Verfahren beschrieben, das zum Betrieb einer Hörvorrichtung (14) in einem Audiodarbietungssystem (1) dient. Die Hörvorrichtung (14) weist dabei einen Breitbandtransmitter (12) sowie einen Schmalbandempfänger (20) auf. Das Audiodarbietungssystem (1) weist eine Mehrzahl von mit einer Schmalband-Übertragung arbeitenden Audiosendern (4) auf, die im bestimmungsgemäßen Einsatzzustand räumlich voneinander beabstandet angeordnet sind, sowie eine Mehrzahl von Breitbandtransmittern (6), die im bestimmungsgemäßen Einsatzzustand räumlich voneinander beabstandet angeordnet sind. Im Rahmen des erfindungsgemäßen Verfahrens wird mittels der Breitbandtransmitter (6, 12) der Hörvorrichtung (14) sowie des Audiodarbietungssystems (6) zunächst eine Größe ermittelt, die charakteristisch für eine Positionierung einer Hörvorrichtung (14) relativ zu den Audiosendern (4) ist. Anhand dieser charakteristischen Größe wird anschließend ein spezifischer der mehreren Audiosender (4) des Audiodarbietungssystems (1) zur Audioübertragung an die Hörvorrichtung (14) ausgewählt.

## Beschreibung

Die Erfindung betrifft ein Verfahren zum Betrieb einer Hörvorrichtung in einem Audiodarbietungssystem. Außerdem betrifft die Erfindung ein solches Audiodarbietungssystem. Des Weiteren betrifft die Erfindung auch eine Hörvorrichtung.

Hörvorrichtungen dienen üblicherweise zur Ausgabe eines Tonsignals an das Gehör des Trägers dieser Hörvorrichtung. Die Ausgabe erfolgt dabei mittels eines Ausgabewandlers, meist auf akustischem Weg über Luftschall mittels eines Lautsprechers (auch als "Hörer" oder "Receiver" bezeichnet). Häufig kommen derartige Hörvorrichtungen dabei als sogenannte Hörhilfegeräte (auch kurz: Hörgeräte) zum Einsatz. Dazu umfassen die Hörvorrichtungen normalerweise einen akustischen Eingangswandler (insbesondere ein Mikrophon) und einen Signalprozessor (auch: "Controller"), der dazu eingerichtet ist, das von dem Eingangswandler aus dem Umgebungsschall erzeugte Eingangssignal (auch: Mikrophonsignal) unter Anwendung mindestens eines üblicherweise nutzerspezifisch hinterlegten Signalverarbeitungsalgorithmus derart zu verarbeiten, dass eine Hörminderung des Trägers der Hörvorrichtung zumindest teilweise kompensiert wird. Insbesondere im Fall eines Hörhilfegeräts kann es sich bei dem Ausgabewandler neben einem Lautsprecher auch alternativ um einen sogenannten Knochenleitungshörer oder ein Cochlea-Implantat handeln, die zur mechanischen oder elektrischen Einkopplung des Tonsignals in das Gehör des Trägers eingerichtet sind. Unter dem Begriff Hörvorrichtungen fallen zusätzlich insbesondere auch Geräte wie z.B. sogenannte Tinnitus-Masker, Headsets, Kopfhörer und dergleichen.

Typische Bauformen von Hörvorrichtungen, insbesondere Hörgeräten, sind Hinter-dem-Ohr- ("BTE"-) und In-dem-Ohr- ("IdO"- oder"ITE"-) Hörvorrichtungen. Diese Bezeichnungen zielen auf die bestimmungsgemäße Trageposition ab. So weisen Hinter-dem-Ohr-Hörvorrichtungen ein (Haupt-) Gehäuse auf, das hinter der Ohrmuschel getragen wird. Hier kann in Modelle unterschieden werden, deren Lautsprecher in diesem Gehäuse angeordnet ist - die Schallausgabe an das Ohr erfolgt dabei üblicherweise mittels eines Schallschlauchs, der im Gehörgang getragen wird - sowie in Modelle, die einen externen Lautsprecher, der im Gehörgang platziert wird, aufweisen. In-dem-Ohr-Hörvorrichtungen weisen hingegen ein Gehäuse auf, das in der Ohrmuschel oder sogar vollständig im Gehörgang getragen wird.

Hörvorrichtungen, insbesondere Hörgeräte, sind häufig dazu eingerichtet, Audiosignale auf nicht-akustischem Weg zu empfangen, bspw. mittels sogenannter Telefonspulen. Diese sind dazu eingerichtet, per induktiver Kopplung übertragene Signale zu empfangen, um diese dann über die Lautsprecher auszugeben. Genutzt wird dies - neben Telefonie - insbesondere auch, um in öffentlichen Räumen Audio-Informationen bereitzustellen und diese dann in einer Hörvorrichtung zu verarbeiten. Dabei kann es sich unter anderem um die Audioübertagung bei einem Vortrag handeln, die neben der klassischen Ausgabe über Lautsprecher teilweise für Hörgeräteträger auch über entsprechende Spulen erfolgen kann, so dass ein Hörgerät diese aufnehmen und entsprechend - insbesondere mit verringerter Störung durch Hintergrundgeräusche - an den Träger ausgeben kann. Bspw. wird dies aber auch genutzt, um in einem Museum sogenannte Audio-Führer für Hörgeräteträger bereitzustellen.

Nachteilig ist hierbei, dass eine induktive, d. h. elektromagnetische, Übertragung über größere Entfernungen, bspw. in Vortrags- oder Museumsräumen, vergleichsweise ineffektiv und störungsbehaftet sein kann.

Der Erfindung liegt die Aufgabe zugrunde, eine Bereitstellung von Audioinformation an einen Hörgeräteträger zu verbessern.

Diese Aufgabe wird erfindungsgemäß gelöst durch ein Verfahren mit den Merkmalen des Anspruchs 1. Außerdem wird diese Aufgabe erfindungsgemäß gelöst durch ein Audiodarbietungssystem mit den Merkmalen des Anspruchs 12. Des Weiteren wird diese Aufgabe erfindungsgemäß gelöst durch eine Hörvorrichtung mit den Merkmalen des Anspruchs 15. Vorteilhafte und teils für sich erfinderische Ausführungsformen und Weiterentwicklungen der Erfindung sind in den Unteransprüchen und der nachfolgenden Beschreibung dargelegt.

Das erfindungsgemäße Verfahren dient zum Betrieb einer Hörvorrichtung in einem Audiodarbietungssystem. Die Hörvorrichtung weist dabei einen Breitbandtransmitter sowie einen Schmalbandempfänger auf. Das Audiodarbietungssystem weist eine Mehrzahl von mit einer Schmalband-Übertragung arbeitenden Audiosendern auf, die im bestimmungsgemäßen Einsatzzustand räumlich voneinander beabstandet angeordnet sind, sowie eine Mehrzahl von Breitbandtransmittern, die im bestimmungsgemäßen Einsatzzustand räumlich voneinander beabstandet angeordnet sind. Im Rahmen des erfindungsgemäßen Verfahrens wird mittels der Breitbandtransmitter der Hörvorrichtung sowie des Audiodarbietungssystems zunächst eine Größe ermittelt, die charakteristisch für eine Positionierung einer Hörvorrichtung relativ zu den Audiosendern ist. Anhand dieser charakteristischen Größe wird anschließend ein spezifischer der mehreren Audiosender des Audiodarbietungssystems zur Audioübertragung an die Hörvorrichtung ausgewählt.

Das erfindungsgemäße Audiodarbietungssystem ist zur Verwendung im Rahmen des hier und im Folgenden beschriebenen Verfahrens eingerichtet und vorgesehen. Insbesondere weist das Audiodarbietungssystem somit die im Rahmen des Verfahrens für das Audiodarbietungssystem beschriebenen körperlichen, optional aber auch die entsprechenden verfahrenstechnischen, Merkmale (und somit auch die sich daraus ergebenden Vorteile) gleichermaßen auf (sowie auch entsprechend umgekehrt das hier und im Folgenden beschriebene Verfahren die Merkmale des Audiodarbietungssystems und dessen vorteilhafter Ausgestaltungen). Das Audiodarbietungssystem weist also die Mehrzahl der mit (vorzugsweise digitaler) Schmalband-Übertragung arbeitenden Audiosendern (optional auch englisch als "audio broadcasting transmitter" bezeichnet) auf, die im bestimmungsgemäßen Einsatzzustand räumlich voneinander beabstandet angeordnet sind. Diese Audiosender sind vorzugsweise dazu eingerichtet, gemeinsam eine oder jeweils einzeln eine separate Audioinformation an einen Funkempfänger (bspw. eine funkübertragungstechnisch gekoppelte Gegenstelle), insbesondere einen Schmalbandempfänger (optional einen Schmalbandtransmitter), zu übertragen (auch: zu "streamen" oder als "Cast" zu übertragen). Das Audiodarbietungssystem weist außerdem die Mehrzahl von Breitbandtransmittern (engl.: "wideband transmitter") auf, die im bestimmungsgemäßen Einsatzzustand räumlich voneinander beabstandet angeordnet sind. Ferner weist das Audiodarbietungssystem einen Controller auf, der mit den Audiosendern und den Breitbandtransmittern datenübertragungstechnisch verbunden ist.

Optional ist dieser Controller dazu eingerichtet, mittels der Breitbandtransmitter die vorstehend beschriebene Größe, die charakteristisch für die Positionierung der Hörvorrichtung relativ zu den Audiosendern ist, zu ermitteln. Weiter optional ist der Controller ist außerdem dazu eingerichtet, anhand dieser charakteristischen Größe wenigstens einen Audiosender zur Audioübertragung (insbesondere eines sogenannten "broadcast") an die Hörvorrichtung auszuwählen.

Das Audiodarbietungssystem ist somit vorzugsweise dazu eingerichtet, Audioinformation mittels zumindest eines von mehreren räumlich verteilten Audiosendern "auszustrahlen", so dass eine (optional explizit, d. h. mittels einer Art "handshake", gekoppelte) Gegenstelle, insbesondere die Hörvorrichtung, diese ausgestrahlte Audioinformation empfangen und insbesondere an eine die Hörvorrichtung nutzende Person ("Hörgeräteträger") ausgeben kann.

Verfahrensgemäß wird mittels der Breitbandtransmitter eine Positionierungsinformation für die Hörvorrichtung relativ zu den Audiosendern ermittelt und in Abhängigkeit von dieser ausgewählt, von welchem Audiosender die Hörvorrichtung die (entsprechend zugeordnete) Audioübertragung empfangen soll.

Durch die erfindungsgemäße, positionsabhängige Auswahl des Audiosenders - und somit gegebenenfalls auch dessen spezifischer Audioinformation - kann eine vergleichsweise effektive Übertragung ermöglicht werden, da die Audioinformation nicht räumlich großflächig ausgestrahlt werden braucht, sondern näherungsweise individualisiert ausgegeben werden kann.

In einer bevorzugten Verfahrensvariante wird der Schmalbandempfänger der Hörvorrichtung (insbesondere nach oder in Abhängigkeit der vorstehend beschriebenen Auswahl) zur Aufschaltung auf eine ausgestrahlte Audioübertragung des spezifischen Audiosenders angesteuert. Hierzu wird zweckmäßigerweise der Hörvorrichtung auch eine Information (bspw. mittels der Breitbandtransmitter des Audiodarbietungssystems) bereitgestellt, anhand derer der ausgewählte Audiosender erkannt werden kann. Bspw. wird im Rahmen dieser Information der Hörvorrichtung bspw. ein Identifikator für denjenigen Audiosender übergibt, mit dem die Hörvorrichtung zur Audioübertragung koppeln oder auf dessen Audioübertragung die Hörvorrichtung, konkret deren Schmalbandempfänger, aufschalten soll. Dieser Identifikator wird zweckmäßigerweise von dem jeweiligen Audiosender in dessen Audioübertragung mit ausgegeben, bspw. als eine Art Metainformation.

In einer bevorzugten Verfahrensvariante wird als die charakteristische Größe ein Abstand der Hörvorrichtung zu den Audiosendern, insbesondere zu jedem der Audiosender, ermittelt. Anschließend wird derjenige Audiosender, der der Hörvorrichtung nächstgelegen ist, zur Audioübertragung an die Hörvorrichtung ausgewählt. Dadurch können einerseits Übertragungsleistungen auf Seiten der Audiosender möglichst geringgehalten werden, da Sendereichweiten gegenüber einer Übertragung an eine unbekannte Position eines Empfängers gegebenenfalls reduziert werden können. Optional können so auch nur spezifische Audiosender aktiv sein, in deren Reichweite oder "Sendebereich" aktuell eine Hörvorrichtung (oder zumindest ein Schmalbandempfänger eines vergleichbaren Geräts) anwesend ist. Auch letzteres trägt zu einer verringerten Leistungsaufnahme bei. Insbesondere kann ein solches Vorgehen aber bspw. in einem Museum von Vorteil sein. Das Audiodarbietungssystem kann hierbei eine Ausgabeschnittstelle für einen sogenannten Audioguide darstellen, mittels dessen Informationen über einzelne Ausstellungsstücke an entsprechende Empfänger ausgegeben werden können. Die Hörvorrichtung kann hierbei eine persönliche Ausrüstung einer Person sein, bspw. deren Hörhilfegerät, aber grundsätzlich auch ein vom Museum gestelltes Kopfhörerset.

Ein Audiosender kann dabei bspw. genau einem Ausstellungsstück zugeordnet sein und eine Information zu diesem Ausstellungsstück ausgeben. Anhand des vorstehend sowie nachfolgend beschriebenen Vorgehens kann also vorteilhafterweise erfasst werden, vor welchem Ausstellungsstück sich die Person aktuell befindet und die Audioübertragung des entsprechenden Audiosenders ausgewählt werden. Eine manuelle Auswahl einer Audioübertragung für dieses Ausstellungsstück kann somit entfallen. Ebenso können aber auch spezifische Informationen in öffentlichen Räumlichkeiten, bspw. einem Flughafen oder Bahnhof, mittels des nächstgelegenen Audiosenders an einen Passagier ausgegeben werden, bspw. eine Fahrplaninformation, eine Information zu einer Boardingzeit an einem spezifischen Gate oder dergleichen. Für dieses Vorgehen ist zweckmäßigerweise der Abstand zwischen den Breitbandtransmittern des Audiodarbietungssystems und dessen Audiosendern bekannt.

Optional wird auch auf die Position der Hörvorrichtung im Raum (bspw. in einem Ausstellungsraum des Museums) geschlossen, indem einzelne Abstandswerte zwischen der Hörvorrichtung und wenigstens zwei, vorzugsweise aber mehr als zwei, Breitbandtransmittern des Audiodarbietungssystems geschlossen wird. Anhand dieser mehreren Abstandswerte kann die Position der Hörvorrichtung als Schnittpunkt der durch jeden Abstandswert beschriebenen Kreise um den jeweiligen Breitbandtransmitter des Audiodarbietungssystems bestimmt werden. Drei Abstandswerte genügen dabei regelmäßig für eine eindeutige Bestimmung des Position. In diesem Fall ist auch die jeweilige Position der Audiosender im entsprechenden Raum bekannt, so dass durch einen Positionsvergleich die Anordnung der Audiosender relativ zur Hörvorrichtung ermittelt werden kann.

Bevorzugt sind die Audiosender zur Audioübertragung nach der Auracast (insbesondere mit der Marke "Auracast Broadcast Audio" bezeichnet) Audioübertragungstechnik eingerichtet. Diese beruht insbesondere auf dem Bluetooth Low Energy Funkstandard, der eine Schmalband-Übertragung im Band von 2,4 GHz verwendet, insbesondere aufgeteilt auf 40 Kanäle mit 2 MHz Breite. Auracast ist dabei konkret ein "broadcasting" Protokoll, das also insbesondere einer Vielzahl von potentiell unbekannten Empfängern ein Informationssignal zur Verfügung stellt.

Weiter bevorzugt wird als Schmalbandempfänger der Hörvorrichtung entsprechend ein nach dem Bluetooth Funkstandard arbeitender Empfänger, insbesondere ein "Transceiver" (der somit zusätzlich auch zum Senden eingerichtet ist, auch als "Transmitter" oder als "Bluetooth-Modul" bezeichnet), herangezogen.

In einer bevorzugten Ausführung sind die Breitbandtransmitter als Ultrabreitbandtransmitter (kurz: UWB-Transmitter für "ultra wide band") ausgebildet (d. h. werden in einer entsprechenden Verfahrensvariante solche UWB-Transmitter herangezogen). Diese arbeiten mit einer Frequenzbreite von wenigstens 500 MHz und/oder von wenigstens 20% einer Mittenfrequenz. Als Mittenfrequenz ist dabei insbesondere der Mittelwert aus oberer und unterer Grenzfrequenz des entsprechenden Frequenzbandes zu verstehen. Im Gegensatz dazu kommen bei herkömmlichen für derartige Einsatzzwecke vorgesehenen Funktechniken (Schmalband-Funk oder Schmalband-Kommunikation) deutlich schmälere, insbesondere weniger als die Hälfte bemessende Frequenzbreiten zur Anwendung (WLAN bspw. mit maximal etwa 160 MHz im 5 GHz-Band, ansonsten weniger als 100 MHz; Bluetooth mit ebenfalls unter 100 MHz). Außerdem ist der UWB-Transmitter vorzugsweise dazu eingerichtet, lediglich Einzel-Signalpulse (vorzugsweise im Bereich von weniger als 10 ns, teilweise im Bereich um 1 ns) zu senden. Hierbei kommt im Gegensatz zur herkömmlichen Funktechnik (insbesondere WLAN und Bluetooth) keine Modulation eines Trägersignals, konkret dessen Trägerfrequenz, zum Einsatz, sondern insbesondere sogenannte Pulsmodulationstechniken.

In einer vorteilhaften Verfahrensvariante wird die charakteristische Größe anhand einer Signallaufzeitmessung und/oder einer Bestimmung eines Ankunftswinkels (bspw. eines seitens des Breitbandtransmitters der Hörvorrichtung oder umgekehrt gesendeten Signals) ermittelt. Dies hat den Vorteil, dass sich hierdurch eine vergleichsweise hohe Ortsauflösung, d. h. eine hohe Präzision der Position bzw. auch des Abstands der Hörvorrichtung zum jeweiligen Audiosender erzielen lässt. Der Ankunftswinkel wird dabei insbesondere mittels zweier dem jeweiligen Breitbandtransmitter zugeordneter (Empfangs-) Antennen ermittelt.

Vorzugsweise werden die Breitbandtransmitter des Audiodarbietungssystems als sogenannte Ankerpunkte herangezogen. Diese sind dazu eingerichtet, ein Ping-artiges Signal des Breitbandtransmitters der Hörvorrichtung zu empfangen, das dann wiederum zur Ermittlung der Position der Hörvorrichtung, bzw. der charakteristischen Größe, herangezogen wird.

Zweckmäßigerweise werden die Breitbandtransmitter des Audiodarbietungssystems (optional via eine Ansteuerung durch dessen Controller), insbesondere eine jeweils zugeordnete interne Uhr, untereinander synchronisiert. Dadurch kann die vorstehend beschriebene Signallaufzeitmessung besonders präzise erfolgen, da so Signallaufzeitunterschiede zwischen den einzelnen Breitbandtransmittern genauer erfasst werden können. Optional sind die Breitbandtransmitter dazu per Datenkabel verbunden. Optional kann diese Synchronisation aber auch funkbasiert erfolgen. Optional kann eine interne Uhr des Controllers als Referenz herangezogen werden, insbesondere also der Controller die Breitbandtransmitter dazu triggern, auf dessen interne Uhr zu synchronisieren.

In einer weiteren zweckmäßigen Verfahrensvariante werden seitens des Breitbandtransmitters der Hörvorrichtung Signale zum Empfang durch die Breitbandtransmitter des Audiodarbietungssystems (bspw. die vorstehend genannten Ping-artigen Signale) gesendet. Anhand des Empfangs dieser Signale durch die Breitbandtransmitter (Ankerpunkte) wird dann auf eine Bewegung der die Hörvorrichtung tragenden Person geschlossen. Insbesondere wird hierbei an einem zeitlichen Verlauf der vorstehend beschriebenen Abstandswerte oder Ankunftswinkel auf diese Bewegung geschlossen. Unter der Annahme, dass die Person die Hörvorrichtung am Kopf trägt und der Breitbandtransmitter der Hörvorrichtung dabei ebenfalls am Kopf angeordnet ist, kann aufgrund der präzisen Ortsauflösung bei der Bestimmung der für die Position der Hörvorrichtung charakteristischen Größe auch auf eine Kopfbewegung (insbesondere anhand des zeitlichen Verlaufs der Größe bzw. Position der Hörvorrichtung) und/oder Ausrichtung des Kopfs der Person geschlossen werden. Aus dieser Information kann wiederum auf eine Blickrichtung und somit auf ein räumliches Interesse der Person geschlossen werden. In einer optionalen Ausgestaltung der Erfindung wird der Inhalt der Audioübertragung an diese Information angepasst (d. h. verändert), um so eine räumliche Wahrnehmung zu verbessern. Beispielsweise kann dies in einem Kino oder auch bei einem TV-Gerät genutzt werden, um bei einem Blick der Person zur Seite Geräusche, die als aus der Szene auf der Leinwand kommend empfunden werden sollen, auch als solche zu empfinden und nicht bei einer Kopfdrehung stets den gleichen akustischen Eindruck zu erhalten. Insbesondere kann hierzu die Balance eines Stereo- oder Surround-Signals in Abhängigkeit von der Blickrichtung verändert werden.

Alternativ oder zusätzlich zu der vorstehend beschriebenen Anpassung des Inhalts der Audioübertragung wird eine Überwachung der die Hörvorrichtung tragenden Person ermöglicht und auch durchgeführt. Anhand der wie vorstehend beschrieben ermittelten Bewegung der Hörvorrichtung wird dabei auf einen Sturz der Person geschlossen, bspw. wenn ein Bewegungsumfang in vertikaler Richtung der Körpergröße der Person entspricht oder zumindest ein vorgegebenes Maß (bspw. mehr als 1,2 oder 1,4 Meter) überschreitet, so dass ein Hinsetzen der Person vergleichsweise unwahrscheinlich ist. Wird auf diese Weise seitens des Controllers auf einen Sturz der Person geschlossen, wird optional ein Notruf abgesetzt und/oder eine Anfrage durch die Hörvorrichtung ausgegeben. Sowohl der Notruf als auch die Anfrage kann dabei durch die Hörvorrichtung selbst abgesetzt bzw. ausgegeben werden. Alternativ kann dies grundsätzlich aber auch durch das Audiodarbietungssystem erfolgen.

In einer zweckmäßigen Ausgestaltung ist jedem Audiosender (insbesondere genau) ein Breitbandtransmitter (Ankerpunkt) zugeordnet. Vorzugsweise ist der jeweils zugeordnete Breitbandtransmitter dem Audiosender auch räumlich zugeordnet. Zweckmäßigerweise ist in diesem Fall der jeweilige Breitbandtransmitter näher zu dem entsprechenden Audiosender angeordnet als zu dem nächstgelegenen der anderen Audiosender. Dadurch wird eine besonders einfache Zuordnung des nächstgelegenen Audiosenders zu der Hörvorrichtung ermöglicht.

Optional können die vorstehend genannten Ankerpunkte aber auch unabhängig von dem Audiodarbietungssystem vorhanden sein, bspw. auch in ein Hausüberwachungssystem eingebunden sein.

Das vorstehend beschriebene Verfahren wird gemäß einer optionalen Ausgestaltung zumindest zum Großteil durch den Controller des Audiodarbietungssystems ausgeführt. In diesem Fall wird die getroffene "Auswahl" des Audiosenders an die Hörvorrichtung übermittelt, wobei seitens der Hörvorrichtung "nur noch" diese Auswahl umgesetzt wird, indem auf die entsprechende Audioübertragung aufgeschaltet wird (d. h. insbesondere der Schmalbandempfänger zum Empfang der Audioübertragung des ausgewählten Audiosenders angesteuert wird, vorzugsweise auf Basis des vorstehend genannten Identifikators). Insbesondere wird die charakteristische Größe also auf der Seite des Audiodarbietungssystems auf Basis des Empfangs eines Signals seitens des Breitbandtransmitters der Hörvorrichtung ermittelt. Alternativ wird das vorstehend beschriebene Verfahren zumindest zum Großteil auf der Seite der Hörvorrichtung, bspw. mittels eines Signalprozessors, vorzugsweise aber auf einem zugeordneten (und datenübertagungstechnisch mit der Hörvorrichtung gekoppelten) Gerät, bspw. einem Smartphone, durchgeführt. Beispielsweise kann in diesem Fall auf der Seite des Audiodarbietungssystem ebenfalls die charakteristische Größe ermittelt werden und diese an die Hörvorrichtung zur weiteren Auswertung übertragen werden. Oder die Breitbandtransmitter des Audiodarbietungssystems übertragen als Antwort auf das (Ping-artige) Signal der Breitbandtransmitters der Hörvorrichtung ein eigenes Signal, das bspw. die - vorzugsweise synchronisierte - Sendeuhrzeit, und vorzugsweise auch den Identifikator des zugeordneten Audiosenders, enthält, so dass auf der Seite der Hörvorrichtung (bspw. im Smartphone) anhand dieser Antwort die charakteristische Größe bestimmt und hieraus der entsprechende Audiosender ausgewählt werden kann.

Die erfindungsgemäße Hörvorrichtung weist wie vorstehend beschrieben den zum Empfang von (insbesondere seitens der Audiosender ausgegebenen) Audiosignalen (also der Audioübertragung) eingerichteten Schmalbandempfänger (bspw. das Bluetooth Modul), den Breitbandtransmitter sowie einen Signalprozessor auf. Der letztere ist dabei datenübertragungstechnisch mit dem Schmalbandempfänger und dem Breitbandtransmitter gekoppelt. Die Hörvorrichtung ist außerdem, vorzugsweise mittels einer entsprechenden Programmierung des Signalprozessors zur Verwendung mit dem vorstehend beschriebenen Audiodarbietungssystem eingerichtet.

Der Breitbandtransmitter der Hörvorrichtung ist dabei zweckmäßigerweise zur Kommunikation mit den Breitbandtransmittern des Audiodarbietungssystems eingerichtet, insbesondere also im gleichen Frequenzband zu senden und empfangen (sowie insbesondere auch den gleichen Funkstandard zu verwenden).

In einer optionalen Ausgestaltung ist der Signalprozessor der Hörvorrichtung dazu eingerichtet, das vorstehend beschriebene Verfahren selbsttätig durchzuführen. Dazu werden dem Signalprozessor, insbesondere mittels der Breitbandtransmitter, gegebenenfalls erforderliche Informationen, insbesondere die vorstehend beschriebenen Identifikatoren der Audiosender, optional die bspw. seitens des Controllers des Audiodarbietungssystems ermittelten Signallaufzeiten, Ankunftswinkel und/oder auch die Abstandswerte oder Position der Hörvorrichtung relativ zu den Audiosendern übermittelt. Vorzugsweise ist die Hörvorrichtung aber im bestimmungsgemäßen Betrieb mit einem Smartphone oder einem anderen Gerät datenübertagungstechnisch gekoppelt, das eine installierte Fernsteuerungssoftware für die Hörvorrichtung aufweist, wobei die vorgenannten Informationen dieser Fernsteuerungssoftware bereitgestellt werden. Diese Fernsteuerungssoftware ist dabei dazu eingerichtet, das vorstehend beschriebene Verfahren durchzuführen, insbesondere den entsprechenden Audiosender auszuwählen und diese Information an die Hörvorrichtung zu übertragen. Diese Fernsteuerungssoftware (insbesondere ein diese ausführender (Mikro-) Prozessor) ermittelt also anhand des Signalaustauschs zwischen den Breitbandtransmittern der Hörvorrichtung und des Audiodarbietungssystems die charakteristische Größe und wählt anhand dieser wiederum den entsprechenden Audiosender aus.

Charakteristisch bedeutet hier insbesondere, dass die charakteristische Größe eine quantitative Information über die Position der Hörvorrichtung relativ zu den Breitbandtransmittern beinhaltet, so dass sich die Position, zumindest aber der Abstand eindeutig aus der charakteristischen Größe ablesen lässt. Die charakteristische Größe kann hierbei die Größe (bspw. den Wert) der jeweiligen Position (insbesondere des Abstands) unmittelbar angeben. Bei der charakteristischen Größe kann es sich aber auch um eine Größe handeln, die zu der anzuzeigenden Position bzw. des Abstands direkt oder indirekt proportional ist oder zu dieser bzw. diesem in einer nicht-linearen, beispielsweise einer logarithmischen, exponentiellen oder polynomialen (also quadratischen, kubischen, etc.) Beziehung steht.

Der Controller des Audiodarbietungssystem sowie der Signalprozessor können im Rahmen der Erfindung als nicht-programmierbare elektronische Schaltung ausgebildet sein. Der Controller bzw. Signalprozessor kann im Rahmen der Erfindung allerdings auch durch einen Mikrocontroller gebildet sein, in dem die vorstehend beschriebene Funktionalität in Form eines Softwaremoduls implementiert ist. Der Prozessor des Smartphones oder des externen Geräts ist vorzugsweise als Mikrocontroller ausgebildet.

Die Konjunktion "und/oder" ist hier und im Folgenden insbesondere derart zu verstehen, dass die mittels dieser Konjunktion verknüpften Merkmale sowohl gemeinsam als auch als Alternativen zueinander ausgebildet sein können.

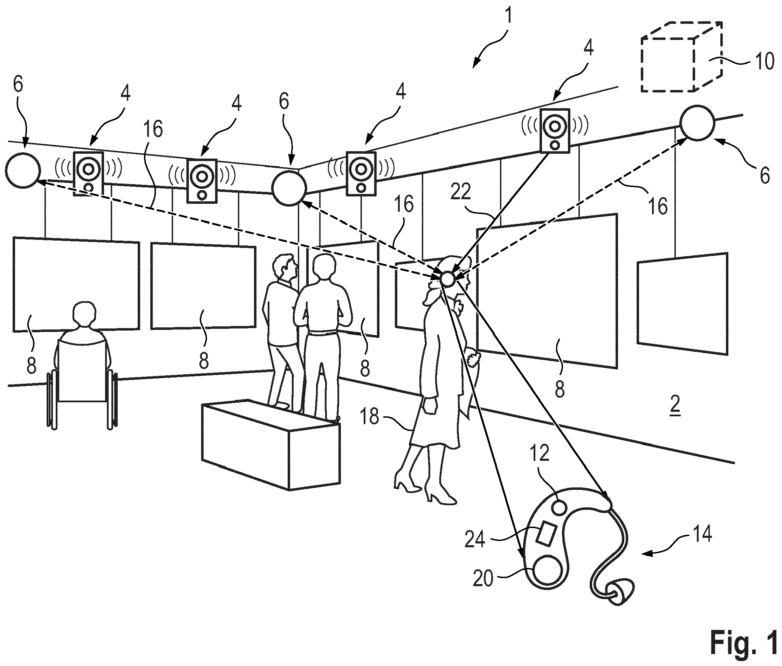

Nachfolgend wird ein Ausführungsbeispiel der Erfindung anhand einer Zeichnung näher erläutert. Darin zeigt die einzige Figur Fig. 1 schematisch ein Audiodarbietungssystem in einem bestimmungsgemäßen Einsatzzustand.

Fig. 1 zeigt ein Audiodarbietungssystem 1, das in einem Ausstellungsraum 2 installiert ist. Das Audiodarbietungssystem 1 weist mehrere, hier konkret vier, Audiosender 4 auf, die dazu eingerichtet sind, nach dem Bluetooth Low Energy Funkstandard zu arbeiten, konkret für "Auracast Broadcast Audio" (eingetragene Marke) eingerichtet sind. Des Weiteren weist das Audiodarbietungssystem 1 mehrere, hier konkret drei, Breitbandtransmitter - im Folgenden als "UWB-Anker 6" bezeichnet - auf. Die Audiosender 4 und die UWB-Anker 6 sind mit Abstand zueinander im Ausstellungsraum 2 verteilt. Die Audiosender 4 sind dabei räumlich jeweils einem ausgestellten Bild 8 zugeordnet. Die Audiosender 4 sind dazu eingerichtet, eine Audioübertragung auszustrahlen, deren Inhalt das jeweilige Bild 8 betrifft. So kann ein Audioguide für den Ausstellungsraum 2 realisiert werden. Die Audiosender 4 sowie die UWB-Anker 6 sind mit einem Controller 10 (kabel- oder funkgebunden) verbunden, um Daten auszutauschen.

Die UWB-Anker 6 dienen konkret dazu, ein Ping-artiges Signal eines Breitbandtransmitters - im Folgenden kurz als UWB-Transmitter 12 - einer Hörvorrichtung 14 aufzunehmen, d. h. zu empfangen, und daraufhin mit diesem einen Signalaustausch einzugehen (s. bidirektionale, strichlinierte Pfeile 16). Im vorliegenden Beispiel trägt eine Person 18 diese Hörvorrichtung 14. Anhand dieses Signalaustauschs mit mehreren der UWB-Anker 6 wird einen Abstand zwischen der Hörvorrichtung 14 ermittelt, also der Person 18, und den jeweiligen UWB-Ankern 6. Diese Ermittlung kann bspw. vom Controller 10 des Audiodarbietungssystems 1 durchgeführt werden. Dazu ermittelt der Controller 10 die Signallaufzeit zwischen jedem UWB-Anker 6 und dem UWB-Transmitter 12 als charakteristische Größe für die Relativposition der Hörvorrichtung 14 zu den UWB-Ankern 6 - und damit, die Kenntnis der Anordnung der Audiosender 4 zu den UWB-Ankern 6 vorausgesetzt - auch zu den Audiosendern 4. Die Position der Hörvorrichtung 14 im Ausstellungsraum 2 wird dabei beispielsweise als Schnittpunkt von Kreisen mit den entsprechenden Abstandswerten als Radius um den korrespondierenden UWB-Anker 6 ermittelt.

Hat der Controller 10 die Position der Hörvorrichtung 14 ermittelt, zumindest welcher Audiosender 4 der nächstgelegene zu der Hörvorrichtung 14 und damit zur Person 18 ist, gibt der Controller 10 diese Information gemeinsam mit einem Identifikator für den entsprechenden Audiosender 4 an die Hörvorrichtung 14 aus. Die Hörvorrichtung 14 weist daraufhin einen Schmalbandempfänger 20 zum Empfang der mittels dieses Audiosenders 4 versendeten Audiosignale (durchgezogener, unidirektionaler Pfeil 22) an.

Die Hörvorrichtung 14 weist auch einen Signalprozessor 24 auf. Dieser ist dazu eingerichtet, für die Kommunikation mit den UWB-Ankern 6 und dem entsprechenden Audiosender 4, den UWB-Transmitter 12 bzw. den Schmalbandempfänger 20 entsprechend anzusteuern.

Alternativ kann die vorstehend beschriebene Ermittlung des nächstgelegenen Audiosenders 4 anhand des Signalaustauschs aber auch durch den Signalprozessor 24 oder ein datenübertragungstechnisch mit der Hörvorrichtung 14 gekoppeltes Smartphone (nicht dargestellt) erfolgen. In diesem Fall wertet der Signalprozessor (oder das Smartphone, an das die Hörvorrichtung 14 die entsprechenden Daten überträgt) die von den UWB-Ankern 6 empfangenen Signale aus und ermittelt daraus den jeweiligen Abstand zu diesen. Seitens der UWB-Anker 6 wird in diesem Fall ein Identifikator für die jeweiligen Audiosender 4 übermittelt, so dass der Signalprozessor 24 (oder das Smartphone) selbst den nächstgelegenen Audiosender 4 auswählen und auch auf dessen Audioübertragung aufschalten kann.

Der Gegenstand der Erfindung ist nicht auf das vorstehend beschriebene Ausführungsbeispiel beschränkt. Vielmehr können weitere Ausführungsformen der Erfindung von dem Fachmann aus der vorstehenden Beschreibung abgeleitet werden.

### Bezugszeichenliste

- 1: Audiodarbietungssystem
- 2: Ausstellungsraum
- 4: Audiosender
- 6: UWB-Anker
- 8: Bild
- 10: Controller
- 12: UWB-Transmitter
- 14: Hörvorrichtung
- 16: Pfeil
- 18: Person
- 20: Schmalbandempfänger
- 22: Pfeil
- 24: Signalprozessor

## Patentansprüche

1. Verfahren zum Betrieb einer Hörvorrichtung (14), die einen Breitbandtransmitter (12) sowie einen Schmalbandempfänger (20) aufweist, in einem Audiodarbietungssystem (1), das eine Mehrzahl von mit einer Schmalband-Übertragung arbeitenden Audiosendern (4), die im bestimmungsgemäßen Einsatzzustand räumlich voneinander beabstandet angeordnet sind, sowie eine Mehrzahl von Breitbandtransmittern (6), die im bestimmungsgemäßen Einsatzzustand räumlich voneinander beabstandet angeordnet sind, aufweist, wobei verfahrensgemäß
mittels der Breitbandtransmitter (6, 12) der Hörvorrichtung (14) sowie des Audiodarbietungssystems (1) eine Größe, die charakteristisch für eine Positionierung einer Hörvorrichtung (14) relativ zu den Audiosendern (4) ist, ermittelt wird, und anhand dieser charakteristischen Größe ein spezifischer der mehreren Audiosender (4) des Audiodarbietungssystems (1) zur Audioübertragung an die Hörvorrichtung (14) ausgewählt wird.

2. Verfahren nach Anspruch 1,
wobei der Schmalbandempfänger (20) der Hörvorrichtung (14) zur Aufschaltung auf eine ausgestrahlte Audioübertragung des spezifischen Audiosenders (4) angesteuert wird.

3. Verfahren nach Anspruch 1 oder 2,
wobei als die charakteristische Größe ein Abstand der Hörvorrichtung (14) zu den Audiosendern (4) ermittelt und der der Hörvorrichtung (14) nächstgelegene Audiosender (4) zur Audioübertragung an die Hörvorrichtung (14) ausgewählt wird.

4. Verfahren nach einem der Ansprüche 1 bis 3,
wobei als Schmalbandempfänger (20) der Hörvorrichtung (14) ein nach dem Bluetooth Funkstandard arbeitender Transceiver herangezogen wird.

5. Verfahren nach einem der Ansprüche 1 bis 4,
wobei als jeweiliger Breitbandtransmitter (6, 12) ein Ultrabreitbandtransmitter herangezogen wird.

6. Verfahren nach einem der Ansprüche 1 bis 5,
wobei die charakteristische Größe anhand einer Signallaufzeitmessung und/oder einer Bestimmung eines Ankunftswinkels ermittelt wird.

7. Verfahren nach einem der Ansprüche 1 bis 6,
wobei die Breitbandtransmitter (6) des Audiodarbietungssystems (1) als sogenannte Ankerpunkte herangezogen werden, wobei mittels dieser Ankerpunkte ein Ping-artiges Signal des Breitbandtransmitters (12) der Hörvorrichtung (14) empfangen wird, das zur Positionsbestimmung der Hörvorrichtung (14) herangezogen wird.

8. Verfahren nach einem der Ansprüche 1 bis 7,
wobei zugeordnete interne Uhr der Breitbandtransmitter (6) des Audiodarbietungssystems (1) untereinander synchronisiert werden.

9. Verfahren nach einem der Ansprüche 1 bis 8,
wobei seitens des Breitbandtransmitters (12) der Hörvorrichtung (14) Signale zum Empfang durch die Breitbandtransmitter (6) des Audiodarbietungssystems (1) gesendet werden und wobei anhand des Empfangs dieser Signale auf eine Bewegung der die Hörvorrichtung (14) tragenden Person (18) geschlossen wird.

10. Verfahren nach Anspruch 9,
wobei anhand der mittels der Breitbandtransmitter (6) empfangenen Signale des Breitbandtransmitters (12) der Hörvorrichtung (14) auf eine Kopfbewegung und/oder eine Ausrichtung des Kopfs der die Hörvorrichtung (14) tragenden Person (18) geschlossen wird sowie in Abhängigkeit von der Kopfbewegung bzw. Ausrichtung des Kopfs ein Inhalt der Audioübertragung verändert wird.

11. Verfahren nach Anspruch 9 oder 10,
wobei anhand der ermittelten Bewegung der Person (18) auf einen Sturz der Person (18) geschlossen und bei einem erkannten Sturz einen Notruf abgesetzt und/oder eine Anfrage durch die Hörvorrichtung (14) ausgegeben wird.

12. Audiodarbietungssystem (1) zur Verwendung in einem Verfahren gemäß einem der Ansprüche 1 bis 11, aufweisend
- eine Mehrzahl von mit einer Schmalband-Übertragung arbeitenden Audiosendern (4), die im bestimmungsgemäßen Einsatzzustand räumlich voneinander beabstandet angeordnet sind,
- eine Mehrzahl von Breitbandtransmittern (6), die im bestimmungsgemäßen Einsatzzustand räumlich voneinander beabstandet angeordnet sind, und
- einen Controller (10), der mit den Audiosendern (4) und den Breitbandtransmittern (6) datenübertragungstechnisch verbunden ist.

13. Audiodarbietungssystem (1) nach Anspruch 12,
wobei jedem Audiosender (4) ein Breitbandtransmitter (12) zugeordnet ist.

14. Audiodarbietungssystem (1) nach Anspruch 12 oder 13,
wobei der jeweilige Breitbandtransmitter (12) näher zu dem entsprechenden Audiosender (4) angeordnet ist als zu dem nächstgelegenen der anderen Audiosender (4).

15. Hörvorrichtung (14), aufweisend einen zum Empfang von Audiosignalen eingerichteten Schmalbandempfänger (20), einen Breitbandtransmitter (6) sowie einen Signalprozessor (24), der datenübertragungstechnisch mit dem Schmalbandempfänger (20) und dem Breitbandtransmitter (6) gekoppelt ist, wobei die Hörvorrichtung (14) zur Verwendung in einem Verfahren gemäß einem der Ansprüche 1 bis 11 eingerichtet ist.
